Europäisches Patentamt

European Patent Office

Office européen des brevets

(19)

(11) Veröffentlichungsnummer: **0 180 272 B1**

(12)

# EUROPÄISCHE PATENTSCHRIFT

(45) Veröffentlichungstag der Patentschrift:
18.01.89

(21) Anmeldenummer: 85201641.9

(22) Anmeldetag: 09.10.85

(51) Int. Cl.⁴: **H 01 J 9/44, H 01 F 13/00**

(54) Verfahren zur Erzeugung einer Mehrpoldauermagnetfeldkonfiguration bei der Herstellung einer Elektronenstrahlröhre und Anordnung zur Durchführung dieses Verfahrens.

(30) Priorität: 12.10.84 NL 8403112

(43) Veröffentlichungstag der Anmeldung:
07.05.86 Patentblatt 86/19

(45) Bekanntmachung des Hinweises auf die Patenterteilung:
18.01.89 Patentblatt 89/3

(84) Benannte Vertragsstaaten:
DE FR GB IT

(56) Entgegenhaltungen:
DE-B- 1 215 820
GB-A- 2 000 635

(73) Patentinhaber: N.V. Philips' Gloeilampenfabrieken,
Groenewoudseweg 1, NL-5621 BA Eindhoven (NL)

(72) Erfinder: van Rijsewijk, Lambertus Jacobus J., p/a INT.
OCTROOIBUREAU B.V. Prof. Holstlaan 6, NL-5656 AA
Eindhoven (NL)
Erfinder: Thomas, Mario Lorenzo, p/a INT.
OCTROOIBUREAU B.V. Prof. Holstlaan 6, NL-5656 AA
Eindhoven (NL)
Erfinder: Meijer, Henricus Johannes Jozephus C., p/a
INT. OCTROOIBUREAU B.V. Prof. Holstlaan 6,
NL-5656 AA Eindhoven (NL)
Erfinder: van der Wilk, Ronald, p/a INT.
OCTROOIBUREAU B.V. Prof. Holstlaan 6, NL-5656 AA
Eindhoven (NL)

(74) Vertreter: Koppen, Jan et al, INTERNATIONAAL
OCTROOIBUREAU B.V. Prof. Holstlaan 6, NL-5656 AA
Eindhoven (NL)

**Beschreibung**

Die Erfindung betrifft ein Verfahren zur Erzeugung einer Mehrpoldauermagnetfeldkonfiguration bei der Herstellung einer Elektronenstrahlröhre, bei der im Hals des Kolbens mit einem Elektronenstrahlerzeugungssystem zumindest ein Elektronenstrahl erzeugt wird und eine um diesen Elektronenstrahl angebrachte Konfiguration aus magnetisierbarem Material zur Erzeugung eines Mehrpoldauermagnetfeldes magnetisiert wird durch eine Mehrpolspuleneinheit mit einer Kombination von Strömen zu erregen, in welchem Verfahren mit dieser Mehrpolspuleneinheit ein statisches Mehrpolmagnetfeld erzeugt und die Magnetisierung mit Hilfe eines abklingenden magnetischen Wechselfeldes erzeugt wird, das zunächst das magnetisierbare Material an beiden Seiten der Hysteresekurve bis in die Sättigung steuert.

Die Erfindung bezieht sich gleichfalls auf eine Anordnung zur Durchführung dieses Verfahrens.

Eine derartige Elektronenstrahlröhre kann beispielsweise eine Farbfernsehbildröhre, eine Monochrom-Bildwiedergaberöhre oder eine Oszillographenröhre sein.

Ein derartiges Verfahren und eine derartige Anordnung sind aus der DE-C-28 28 710.7 bekannt.

Bei einer Farbfernsehbildröhre vom «In-Line»-Typ sind drei Elektronenstrahlerzeuger derart im Röhrenhals angeordnet, dass die Achsen der drei Erzeuger im wesentlichen in einer Ebene liegen, während die Achse des mittleren Elektronenstrahlerzeugers nahezu mit der Achse der Bildwiedergaberöhre zusammenfällt. Die zwei äusseren Elektronenstrahlerzeuger liegen symmetrisch in bezug auf den mittleren Erzeuger. Bei einer Farbfernsehbildröhre vom «Delta»-Typ sind drei Elektronenstrahlerzeuger in Dreieckanordnung im Röhrenhals angeordnet. Die Schnittpunkte der Erzeugerachsen mit einer Ebene senkrecht zur Röhrenachse bilden die Eckpunkte eines gleichschenkligen Dreiecks. Solange die von den Erzeugern erzeugten Elektronenstrahlen nicht abgelenkt werden, müssen sowohl bei Röhren vom «In-Line»-Typ als auch vom «Delta»-Typ die drei Elektronenstrahlen in der Mitte des Bildschirmes zusammenkommen (statische Konvergenz). Da jedoch durch Fehler bei der Herstellung der Bildwiedergaberöhre, beispielsweise ein nicht ganz symmetrisches Einschmelzen der Elektronenstrahlerzeuger in bezug auf die Röhrenachse, Abweichungen in der Rasterform, in der Farbreinheit und in der statischen Konvergenz auftreten, sind diese Abweichungen zu korrigieren.

Eine derartige Farbfernsehröhre vom «In-Line»-Typ, bei der dies der Fall ist, ist aus der DE-C-26 11 633.6 bekannt. Dort ist eine Farbfernsehbildröhre beschrieben, in der die Abweichungen durch Magnetisierung eines Ringes aus magnetisierbarem Material korrigiert werden, wodurch sich ein statischer magnetischer Mehrpol um die Bahnen der Elektronenstrahlen bildet. Dieser Ring ist im Röhrenhals oder um den Röhrenhals angeordnet. Bei dem in der DE-C-26 11 633.6 beschriebenen Verfahren wird die Farbfernsehbildröhre in Betrieb gesetzt, wonach Informationen über die Grösse und die Richtung der Konvergenzfehler der Elektronenstrahlen festgestellt werden, mit deren Hilfe die Polarität und die Stärke des magnetischen Mehrpols bestimmt werden, die für die Korrektur der Raster-, Farbreinheit- und Konvergenzfehler erforderlich sind. Die Magnetisierung der Konfiguration, die aus einem Ring, einem Band oder einer Anzahl von Stäben oder Blöcken um die Elektronenbahnen gruppiert bestehen kann, erfolgt auf die eingangs erwähnte Weise, wobei ein Mehrpol durch nur eine Gesamtmagnetisierung erhalten wird.

In der US-Patentschrift US-A-4 424 466 ist eine Oszillographenröhre beschrieben, in der der Ring aus magnetisierbarem Material zum Korrigieren ganz anderer Fehler verwendet wird. Beispielsweise ist der Ring zwischen dem ersten und dem zweiten Ablenkplattensatz, zwischen dem zweiten Ablenkplattensatz und dem Bildschirm oder zwischen der Kathode und dem ersten Ablenkplattensatz angeordnet. Auch können mehr als ein Ring je Röhre verwendet und magnetisiert werden.

Der Erfindung liegt die Aufgabe zugrunde, dieses Verfahren zu verbessern und die dabei erforderliche Anordnung zu vereinfachen.

Diese Aufgabe wird mit einem verbesserten Verfahren eingangs erwähnter Art erfindungsgemäss dadurch gelöst, dass das abklingende magnetische Wechselfeld ein axiales Magnetfeld ist, das im wesentlichen parallel zum Elektronenstrahl oder zu den Elektronenstrahlen verläuft.

Der Erfindung liegt die Erkenntnis zugrunde, dass das Wechselfeld Streukomponenten enthält, die beim bisher benutzten radial gerichteten Wechselfeld einen geringen Konvergenzrestfehler verursachen. Da das Wechselfeld jetzt axial gerichtet ist, also in der Fortpflanzungsrichtung des Elektronenstrahls, ist die Auswirkung dieser Streukomponenten wesentlich geringer. Die Anforderungen, die an die Qualität des Wechselfelds gestellt werden, können dadurch geringer sein. Bei der in der DE-PS 28 28 710.7 beschriebenen Anordnung ist das Wechselfeld radial gerichtet, wodurch störendes Übersprechen in den Spulen der Mehrpolspuleneinheit auftrat. Ein bevorzugtes Verfahren ist daher dadurch gekennzeichnet, dass das Magnetwechselfeld im wesentlichen senkrecht zum Magnetfeld der Mehrpolspuleneinheit verläuft. Das magnetische Übersprechen wird dadurch auf ein Mindestmass reduziert.

Eine Anordnung zur Durchführung des erfindungsgemässen Verfahrens ist dadurch gekennzeichnet, dass diese Anordnung eine Mehrpolspuleneinheit, deren Spulenachsen sich im wesentlichen radial von der Röhrenachse erstrecken, und zumindest eine Wechselfeldspule enthält, deren Achse im wesentlichen mit der Röhrenachse zusammenfällt.

Die Verwendung einer oder zweier axialer Spulen ist viel einfacher als die der bisher üblichen radial gerichteten Spulen, bei denen zum Erhalten eines magnetischen Drehfeldes eine kompliziertere Ansteuerung erforderlich war.

Die Wechselfeldspule kann innerhalb oder auserhalb der Mehrpolspuleneinheit angebracht werden. Die Wechselfeldspule kann – in Fortpflanzungsrichtung des Elektronenstrahls gesehen – vor oder hinter der Mehrpolspuleneinheit angeordnet werden.

Bei einem radial gerichteten magnetischem Wechselfeld wurden Rotationskräfte auf den Strahlerzeuger ausgeübt, die die Positionierung des Erzeugers ungünstig beeinflussen konnten. Diese Beeinflussung tritt bei der Anwendung des erfindungsgemässen Verfahrens nicht mehr auf. Allerdings zeigt es sich bei der Anwendung des Verfahrens, dass eine geringere Streuung der Erzeugerposition in der Röhre auftritt.

Ein weiterer Vorteil ist, dass die Magnetisierungseinheit durch die Benutzung einer axial gerichteten Wechselfeldspule wesentlich kleiner ausgeführt werden kann.

Ausführungsbeispiele der Erfindung werden nachstehend an Hand der Zeichnung näher erläutert. Es zeigen

Fig. 1 einen Längsschnitt durch eine Farbfernsehbildröhre vom «In-Line»-Typ mit einer erfindungsgemässen Magnetisierungsanordnung,

Fig. 2 einen Schnitt der Fig. 1,

Fig. 3 einen anderen Schnitt der Fig. 1 und

Fig. 4a, b, c und d andere Ausführungsbeispiele der Anordnung nach der Erfindung.

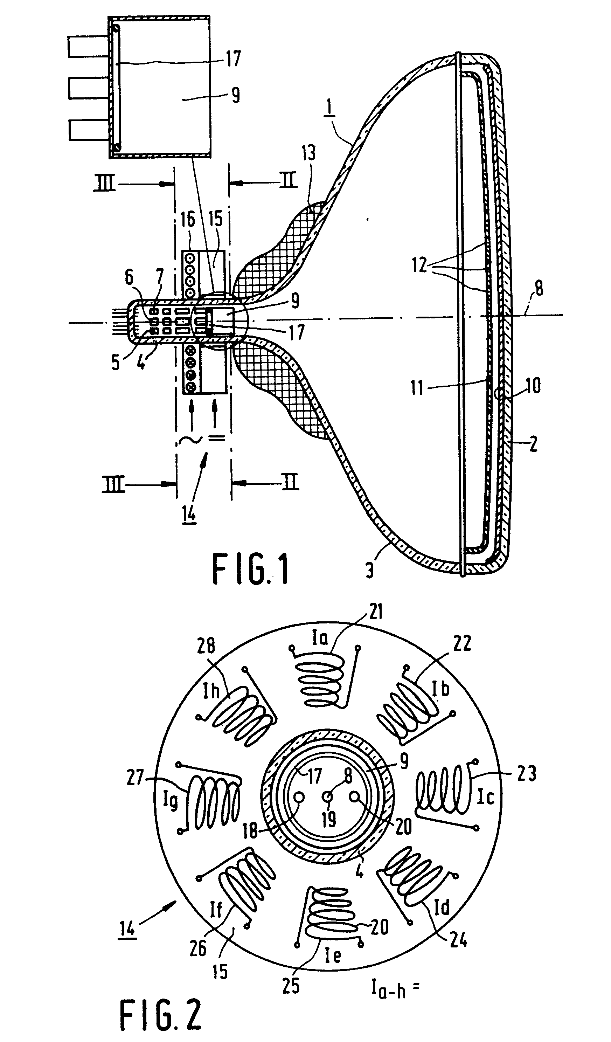

In Fig. 1 ist schematisch eine bekannte Farbfernsehbildröhre vom «In-Line»-Typ im Schnitt dargestellt. In einem Glaskolben 1, der aus einem Bildfenster, einem trichterförmigen Teil 3 und einem Hals 4 besteht, sind in diesem Hals drei Elektronenstrahlerzeuger 5, 6 und 7 angebracht, die drei Elektronenstrahlen erzeugen. Die Achsen der Elektronenstrahlerzeuger liegen in einer Ebene, und zwar der Zeichenebene. Die Achse des mittleren Elektronenstrahlerzeugers 6 fällt mit der Röhrenachse 8 nahezu zusammen. Die drei Elektronenstrahlerzeuger münden in eine Buchse 9, die koaxial im Hals 4 liegt. Das Bildfenster 2 weist an der Innenseite eine Vielzahl von Leuchtstofflinientripeln auf. Jedes Tripel enthält eine Linie aus einem grünleuchtenden Werkstoff, eine Linie aus einem blauleuchtenden Werkstoff und eine Linie aus einem rotleuchtenden Werkstoff. Alle Tripel zusammen bilden den Bildschirm 10. Die Leuchtstofflinien verlaufen senkrecht zur Zeichenebene. Vor dem Bildschirm ist eine Lochmaske 11 angeordnet, in der eine Vielzahl länglicher Öffnungen 12 angebracht ist, durch die die Elektronenstrahlen gehen. Die Elektronenstrahlen werden in horizontaler Richtung (in der Zeichenebene und in vertikaler Richtung senkrecht dazu) von einem Ablenkspulensystem abgelenkt. Die drei Elektronenstrahlerzeuger sind derart montiert, dass ihre Achsen einen spitzen Winkel miteinander bilden. Die erzeugten Elektronenstrahlen fallen dadurch unter diesem Winkel, dem sog. Farbauswahlwinkel, durch die Öffnungen 12 und erreichen je nur Leuchtstofflinien einer einzigen Farbe. Eine Bildröhre hat eine gute statische Konvergenz, wenn die drei Elektronenstrahlen, wenn sie nicht abgelenkt werden, einander nahezu in der Mitte des Bildschirms schneiden. Es zeigt sich jedoch, dass die statische Konvergenz sowie die Rasterform und die Farbreinheit oft nicht richtig sind, was die Folge einer nicht ausreichend genauen Erzeugermontage und/oder Einschmelzung der Elektronenstrahlerzeuger im Röhrenhals sein kann.

Durch eine entsprechende Magnetisierung einer Konfiguration aus magnetisierbarem Material, beispielsweise eines Ringes, können die Fehler in der Konvergenz, in der Farbreinheit und im Raster des dargestellten Bildes zum grössten Teil beseitigt werden. Dies ist ausführlich in der erwähnten DE-C-28 28 710.7 beschrieben.

Eine Magnetisierungsanordnung 14 enthält eine Mehrpolspuleneinheit 15 und eine Wechselfeldspule 16. Die Anordnung 14 ist um eine Konfiguration aus magnetisierbarem Material angebracht, in diesem Fall um einen Ring 17 aus einer Legierung von Fe, Co, V und Cr (unter dem Warenzeichen «Vicalloy» bekannt), der am Boden der Buchse 9 um die Elektronenstrahlen befestigt ist. Es ist klar, dass der Ring 17 auch an anderen Stellen im Röhrenhals oder um den Röhrenhals angebracht werden kann. Statt eines Ringes ist es auch möglich, ein Band oder eine Konfiguration aus Stäben oder Blöcken aus magnetisierbarem Material zu verwenden.

In Fig. 2 ist ein Schnitt der Fig. 1 dargestellt. Im Hals 4 befindet sich die Buchse 9 mit dem Ring 17 am Boden; dieser Ring ist um die Elektronenstrahlen 18, 19, 20 angeordnet. Um den Röhrenhals 4 ist die Magnetisierungseinheit 14 angeordnet, von der hier die Mehrpolspuleneinheit 15 dargestellt ist. Diese Einheit enthält Spulen 21 bis 28 zum Erzeugen des gewünschten Mehrpolfeldes. Ein Mehrpolfeld ist eine Kombination von Zweipolen, Vierpolen, Sechspolen und ggf. Mehrpolen. Abhängig von den durchzuführenden Korrekturen können mit dieser Mehrpolspuleneinheit verschiedene reine Zweipole, Vierpole, Sechspole und ggf. Pole höherer Ordnung und ihre Kombinationen dadurch erzeugt werden, dass die Spulen 21 bis 28 mit geeigneten Gleichströmen (=) jeweils $I_a$ bis $I_n$ durchflossen werden. Die Achsen der Spulen 21 bis 28 erstrecken sich radial zur Röhrenachse 8. Die von diesen Spulen erzeugten Magnetfelder sind daher radial gerichtet.

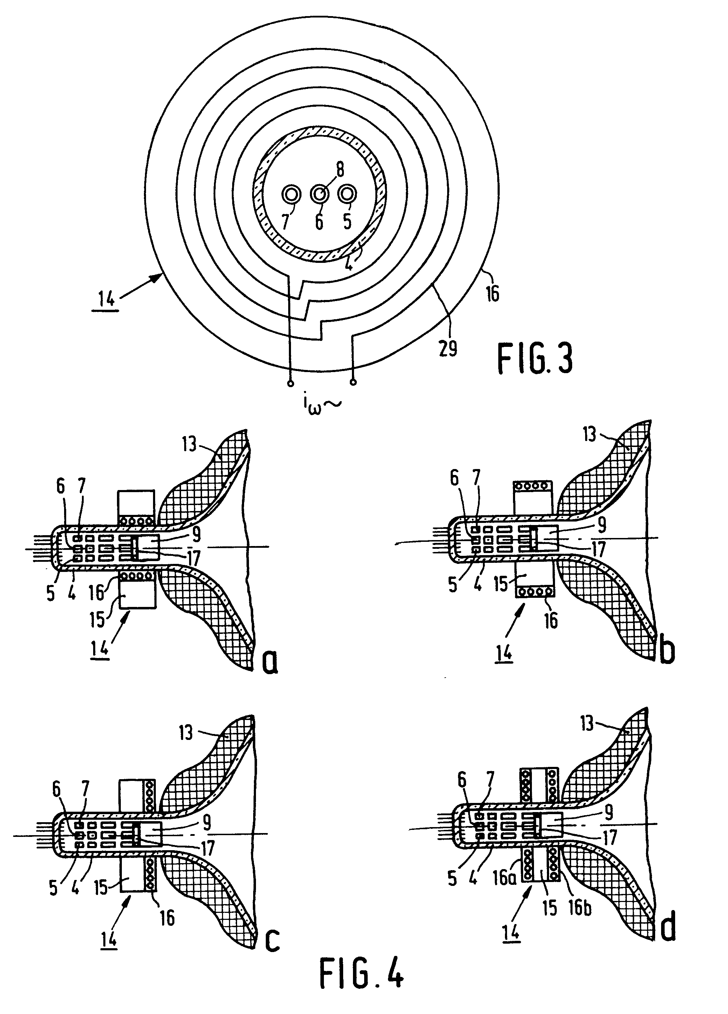

In Fig. 3 ist ein anderer Querschnitt der Fig. 1 dargestellt. Vor der Mehrpolspuleneinheit 15 ist um die Erzeuger 5, 6 und 7 eine Wechselfeldspule 16 angebracht (siehe Fig. 1). Diese Spule enthält nur eine Spule 29, die der abklingende Wechselstrom ($\sim$)$i_w$ durchfliesst, mit dem das abklingende Wechselfeld erzeugt wird. Der Wechselstrom $i_w$ muss in der Magnetisierungsperiode so gross sein, dass das Material des Ringes an beiden Seiten der Hysteresekurve völlig bis in die Sättigung magnetisiert wird. Wenn das Wechselfeld abgeklungen ist, ist der Ring 17 als Mehrpol magnetisiert. Dann ist der von der Mehrpolspuleneinheit erzeugte Mehrpol im Ring magnetisiert und kann die Magnetisierungseinheit entfernt werden. Es wird klar sein, dass, wenn die Magnetisierung nach einem Magnetisierungsschritt noch nicht richtig ist, diese Magnetisierung einmal oder mehrere Male wiederholt werden kann.

Das magnetische Wechselfeld ist axial und im wesentlichen senkrecht zum magnetischen Mehrpolfeld gerichtet. Das Übersprechen des Wechselfeldes in die Spulen der Mehrpolspuleneinheit ist dadurch minimal.

In Fig. 4a bis 4d sind analog der Fig. 1 alternative Ausführungsbeispiele von Magnetisierungsanordnungen nach der Erfindung dargestellt.

In Fig. 4a ist die Wechselfeldspule in der Mehrpolspuleneinheit 15 angeordnet.

In Fig. 4b ist die Wechselfeldspule um die Mehrpolspuleneinheit 15 angeordnet.

In Fig. 4c ist die Wechselfeldspule an der Bildschirmseite an der Mehrpolspuleneinheit angebracht und in Fig. 4d ist die Wechselfeldspule in zwei Teilspulen 16a und 16b aufgeteilt, die an der Mehrpolspuleneinheit 15 angeordnet sind.

Selbstverständlich ist es auch möglich, Kombinationen der in Fig. 4a bis 4d dargestellten Wechselfeldspulen zu verwenden. Wesentlich ist, dass das Wechselfeld axial ist. Die Frequenz des Wechselfeldes kann 50 oder 60 Hz betragen, so dass die Wechselfeldspule(n) direkt aus dem Netz gespeist werden kann (können). Es ist jedoch auch möglich, andere Frequenzen zu verwenden.

Das Verfahren ist nur an Hand eines Schnittes durch eine Farbfernsehbildröhre erläutert. Dieses Verfahren lässt sich jedoch auch beim Magnetisieren einer Konfiguration aus magnetisierbarem Material (beispielsweise eines Ringes) in einer Oszillographenröhre nach der Beschreibung in der US-Patentschrift US-A-4 424 466 oder in einer monochromen Wiedergaberöhre, wie einer DGD-Röhre (Daten-Graphik-Röhre) oder in einer Projektionsfernsehbildröhre verwenden.

## Patentansprüche

1. Verfahren zur Erzeugung einer Mehrpoldauermagnetfeldkonfiguration bei der Herstellung einer Elektronenstrahlröhre, bei der im Hals des Kolbens mit einem Elektronenstrahlerzeugungssystem zumindest ein Elektronenstrahl erzeugt wird und eine um diesen Elektronenstrahl angebrachte Konfiguration aus magnetisierbarem Material zur Erzeugung eines Mehrpoldauermagnetfeldes magnetisiert wird durch eine Mehrpolspuleneinheit mit einer Kombination von Strömen zu erregen, in welchem Verfahren mit dieser Mehrpolspuleneinheit ein statisches Mehrpolmagnetfeld erzeugt und die Magnetisierung mit Hilfe eines abklingenden magnetischen Wechselfeldes erzeugt wird, das zunächst das magnetisierbare Material an beiden Seiten der Hysteresekurve bis in die Sättigung steuert, dadurch gekennzeichnet, dass das abklingende magnetische Wechselfeld ein axiales Magnetfeld ist, das im wesentlichen parallel zum Elektronenstrahl oder zu den Elektronenstrahlen verläuft.

2. Verfahren nach Anspruch 1, dadurch gekennzeichnet, dass das magnetische Wechselfeld im wesentlichen senkrecht zum Magnetfeld der Mehrpolspuleneinheit verläuft.

3. Anordnung zur Durchführung des Verfahrens nach einem der Ansprüche 1 oder 2, dadurch gekennzeichnet, dass diese Anordnung eine Mehrpolspuleneinheit, deren Spulenachsen sich im wesentlichen radial von der Röhrenachse erstrecken, und zumindest eine Wechselfeldspule enthält, deren Achse im wesentlichen mit der Röhrenachse zusammenfällt.

4. Anordnung nach Anspruch 3, dadurch gekennzeichnet, dass die Wechselfeldspule innerhalb oder ausserhalb der Mehrpolspuleneinheit angebracht ist.

5. Anordnung nach Anspruch 3, dadurch gekennzeichnet, dass die Wechselfeldspule, in der Fortpflanzungsrichtung des Elektronenstrahls gesehen, vor und/oder hinter der Mehrpolspuleneinheit angeordnet ist.

## Claims

1. A method of producing a multipolar permanent magnetic field configuration when manufacturing a cathode ray tube in which at least one electron beam ist generated in the neck of the envelope by an electron gun system and around which electron beam magnetic poles are provided which generate a multipolar permanent magnetic field and are formed by magnetizing a configuration of magnetizable material, said configuration being provided around the electron beam and being magnetized by energizing, by means of a combination of currents, a multipole coil unit, in which method a static multipole magnetic field is generated by said multipole coil unit and the magnetization is produced by means of a decaying alternating magnetic field which initially drives the magnetizable material on both sides of the hysteresis curve into saturation, characterized in that the decaying alternating magnetic field is an axial magnetic field which extends substantially parallel to the electron beam or beams.

2. A method as claimed in Claim 1, characterized in that the alternating magnetic field extends substantially perpendicular to the magnetic field of the multipole coil unit.

3. A device for carrying out the method as claimed in any of the Claims 1 or 2, characterized in that said device comprises a multipole coil unit the axes of the coils of which extend substantially radially from the tube axis, and at least one alternating field coil whose axis coincides substantially with the tube axis.

4. A device as claimed in Claim 3, characterized in that the alternating field coil is provided inside or outside the multipole coil unit.

5. A device as claimd in Claim 3, characterized in that the alternating field coil, viewed in the direction of propagation of the electron beam, is placed in front of and/or behind the multipole coil unit.

## Revendications

1. Procédé pour la réalisation d'un tube à rayons cathodiques selon lequel au moins un faisceau électronique est réalisé dans le col de l'enveloppe à l'aide d'un système pour la réalisation d'un faisceau électronique et que des pôles magnétiques sont disposés autour de ce faisceau électronique pour former un champ magnétique permanent multipolaire et sont formés par magnétisation d'une configuration de matériaux magnétisables, la configuration étant disposée autour du faisceau électronique et magnétisée par excitation d'une unité de bobines de champs multipolaire avec une combinaison de courants, procédé selon lequel un champ magnétique multipolaire statique est formé avec ladite unité de bobines de champ multipolaire et la magnétisation est obtenue à l'aide d'un champ alternatif magnétique décroissant qui porte d'abord le matériau magnétisable des deux côtés de la courbe d'hystérésis en saturation, caractérisé en ce que le champ alternatif magnétique décroissant est un champ magnétique axial qui s'étend d'une façon essentiellement parallèle au(x) faisceau(x) d'électrons.

2. Procédé selon la revendication 1, caractérisé en ce que le champ alternatif magnétique est essentiellement perpendiculaire au champ magnétique de l'unité de bobines de champs multipolaire.

3. Dispositif pour la mise en œuvre du procédé selon l'une des revendications 1 ou 2, caractérisé en ce que ce dispositif contient une unité de bobines de champs multipolaire, dont les axes de bobines s'étendent de façon essentiellement radiale à partir de l'axe du tube et au moins une bobine de champ alternatif, dont l'axe coïncide essentiellement avec l'axe du tube.

4. Dispositif selon la revendication 3, caractérisé en ce que la bobine de champ alternatif est disposée à l'intérieur ou à l'extérieur de l'unité de bobines de champs multipolaire.

5. Dispositif selon la revendication 3, caractérisé en ce que vu dans la direction de propagation d'électrons, la bobine de champ alternatif est disposée devant ou derrière l'unité de bobines multipolaires.

**FIG.1**

**FIG.2**

FIG. 3

$i_\omega \sim$

FIG. 4